# EUROPEAN PATENT APPLICATION

(11) **EP 0 729 734 A2**
(43) Date of publication of application: **04.09.1996**
(21) Application number: 96301280.2
(22) Date of filing: 26.02.1996
(51) Int. Cl.: A61F 9/00, H01S 3/131

(54) **Apparatus for laser treatment**

(30) Priority: 28.02.1995 JP 68638/95
(71) Applicant: NIDEK CO., LTD, Gamagori-shi, Aichi 443 (JP)
(72) Inventor: Ota, Yasuo, Gamagori-shi, Aichi 443-01 (JP)
(74) Representative: Blatchford, William Michael

(57) **Abstract**

An apparatus for laser treatment with higher reliability comprises a solid state laser oscillator for emitting pulsed laser beam for treatment in response to the inducing light from an exciting light source, a power supply for supplying energy to the exciting light source, a detector device for detecting output energy of the pulsed laser beam emitted from the solid state laser oscillator, a controller device for controlling energy supplied to the exciting light source in accordance with the results of the detection by the detector device, and a guide optics device for guiding pulsed laser beam to a diseased site.

## Description

### BACKGROUND OF THE INVENTION

### 1. Field of the Invention

The present invention relates to an apparatus for laser treatment, which is used for treatment with laser beam for treatment being irradiated to an eye of a patient.

### 2. Description of the Related Art

It is known that there are some modes of oscillation of an apparatus for laser treatment, such as a continuous laser mode in which the thermal effect of laser coagulates the diseased site to be treated, and a pulse mode in which the impulsive effect destroys or cuts out the diseased tissue. Solid state laser, such as an Nd:YAG laser, is generally allowed to be operated in either the continuous or pulse mode. The mode to be used is selectable according to the purpose.

In the solid state laser for generating laser beam by emitting exciting light from an exciter to a laser medium, a method for the operation in pulse mode is known in which a Q switch (an optical shutter) is provided along with the light path in a laser oscillator to generate giant pulses by taking advantage of the long life of laser upper level specific on the solid state laser. There is also another known mode, a burst mode, for continuously oscillating to generate a plurality of pulses.

However, the solid state laser has disadvantages that the laser output power is affected by the ambient temperature in the operational oscillator. Especially in the pulse laser with the Q switch, the changes in temperature affects to exciting energy necessary for obtaining a stable output power. More specifically, the laser using a saturatable Dye-Q switch has problems that the output pulses may not be generated in a low temperature, and that the number of output pulses may be generated more than a predetermined count in a high temperature.

In addition the solid state laser has disadvantages that the laser output power may vary with temporal changes such as the aged deterioration of the inducing light source.

### SUMMARY OF THE INVENTION

The present invention has been made in view of the above circumstances and has an object to overcome the above mentioned problems and to provide an reliable apparatus for laser treatment, which may compensate for the changes in the operational and ambient temperature-and the aged deterioration of the apparatus and may prevent an accidental engagement so as to output stable laser power.

Additional objects and advantages of the present invention will be set forth in part in the description which follows and in part will be obvious from the description, or may be learned by practice of the present invention. The objects and advantages of the invention may be realized and attained by means of the instrumentalities and combinations particularly pointed out in the appended claims.

To achieve the objects and in accordance with the purpose of the present invention, as embodied and broadly described herein, an apparatus for laser treatment of this invention comprises a solid state laser oscillator having a Q switch, responsive to inducing light from an exciting light source for oscillating pulsed laser beam for treatment; a power supply source for supplying variable energy to the exciting light source; guide optics for guiding into a diseased site the pulsed laser beam emitted from the solid state laser oscillator; a pulsed output detector means for counting the number of output pulses by detecting the pulsed laser beam emitted from the solid state laser oscillator by using a light detector; and a controller means for controlling the amount of energy supplied from the power supply to the exciting light source in accordance with the number of pulses of the pulsed laser beam detected by the detector means such that a predetermined number of pulsed laser beam is emitted.

Another apparatus for laser treatment of this invention comprises a solid state laser oscillator responsive to inducing light from an exciting light source for oscillating pulsed laser beam for treatment, a power supply source for supplying energy to the exciting light source, a detector means for detecting the output power of pulsed laser beam emitted from the solid state laser oscillator, a controlling means for controlling energy supplied to the exciting light source on the basis of the results of the detection by the detector means, and a guide optics for guiding pulsed laser beam to a diseased site.

The apparatus further comprises a temperature detecting means for detecting at least one of the temperature within the solid state laser oscillator and the temperature of the surrounding ambient of the apparatus, the controlling means controlling energy supplied to the exciting light source in accordance with the results of the detection by the temperature detecting means.

The solid state laser oscillator is a solid state laser oscillator having a Q switch.

The apparatus further comprises an irradiation condition setting means for presetting condition of irradiation of pulsed laser beam, and an oscillating test means for testing oscillation of pulsed laser beam from the laser oscillator at least either at the time of starting up of the apparatus or at the time of condition presetting with the irradiation condition setting means.

The apparatus further comprises an attenuator means for attenuating pulsed laser beam, the detector means having two set of detecting means for respectively detecting the laser output power in the attenuator means at the side of the solid state laser oscillator and at the side of an eye to be operated.

In accordance with the present invention, an apparatus performs self diagnosis by detecting oscillation laser output in order to compensate for the output for the next laser oscillation, whereby malfunction caused by changes with time such as changes in temperature and aged deterioration of the oscillator light source may be prevented from occurring and a stable laser output can be obtained any time.

Also, the apparatus may automatically compensate itself for a sudden increase in temperature, and may notify the operator of some necessary measures.

In addition, the apparatus can be easily determined where in the apparatus occurs malfunction.

### BRIEF DESCRIPTION OF THE DRAWING

Other features and advantages of the invention will be apparent from the following description taken in connection with the accompanying drawings, in which:

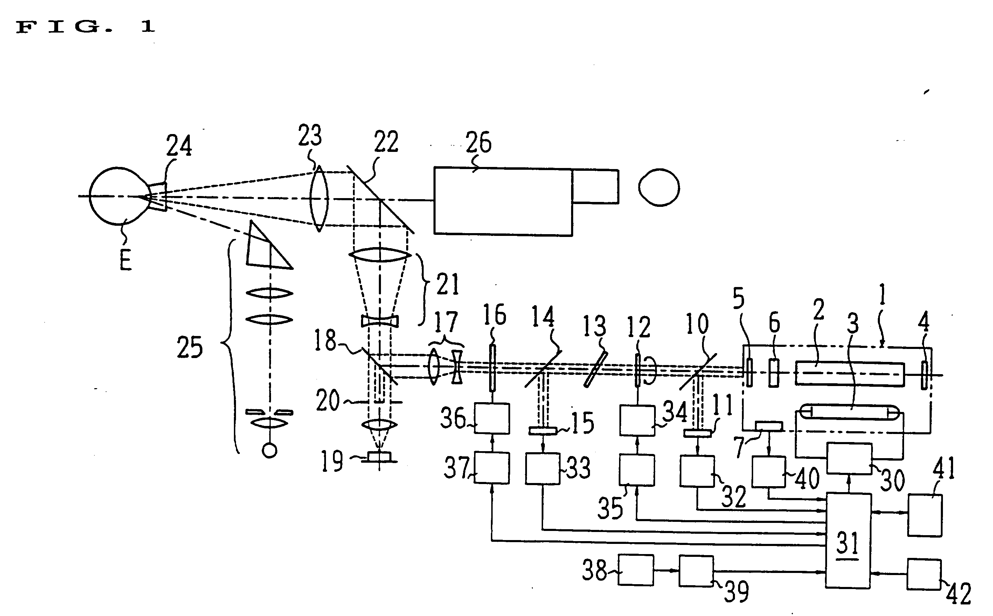

Fig. 1 shows an overview of the optics and control system of an apparatus of a preferred embodiment according to the present invention.

### DETAILED DESCRIPTION OF THE PREFERRED EMBODIMENTS

An preferred embodiment according to the present invention will be described below with reference to the accompanying drawing.

### (Optics)

Referring to Fig. 1, which shows an overview of the optics and control system of an apparatus of the embodiment according to the invention, reference numeral 1 designates a solid state YAG laser oscillator generating laser beam (dominant wavelength: 1,064 nm). The YAG laser oscillator 1 comprises an Nd:YAG rod 2, a flash lamp 3 for providing induced light to the Nd:YAG rod 2, a reflector mirror 4, an output mirror 5, a Dye-Q switch 6 of saturatable dye, provided between the output mirror 5 and the Nd:YAG rod 2. When the Nd:YAG rod 2 is induced by the light of the flash lamp 3, the energy level of the Nd:YAG rod 2 increases, and the Dye-Q switch is decolorized automatically when the energy level of the Nd:YAG rod 2 reaches to a certain predetermined level. When the decolorization saturates, the Dye-Q switch will become transparent, so the Q value of the oscillator will attain to its maximum level, thereby the oscillator starts to oscillate to release the energy stored in the Nd:YAG rod 2 at once to emit giant pulse laser beam from the YAG laser oscillator 1.

Reference numeral 7 designates a sensor for detecting the operational temperature within the YAG laser oscillator 1. 10 is a beam splitter. Part of laser beam reflected by the beam splitter 10 is incident to a light detector 11, which detects the output energy of the laser beam emitted from the YAG laser oscillator 1 based on the received amount of light. 12 is a half-wavelength plate for rotating the direction of polarization of laser beam, and 13 is a polarizing filter placed at the Brewster angle. The half-wavelength plate 12 is rotated by a pulse motor 34 for attenuating the amount of emitted laser beam in combination with the polarizing filter 13.

Reference numeral 14 is another beam splitter for guiding part of laser beam attenuated by the polarizing filter 13 to a light detector 15. The light detector 15 detects the energy of the laser beam passing through the polarizing filter 13 based on the amount of received light.

Reference numeral 16 is a safety shutter, which serves to intercept laser beam in a predetermined case for example of an oscillation test (as explained below) or occurrence of an abnormal event. After passing through the safety shutter 16, the laser beam bundle is expanded by an expanding lens 17, then made coaxial with aiming light (dominant wavelength: 685 nm, more preferably 635 nm or 650 nm) from a visible light semiconductor laser 19 by using a dichroic mirror 18. The aiming light emitted from the visible light semiconductor laser 19 is split by an aperture 20 having two openings into two beams.

Reference numeral 21 is another expander lens for expanding laser bundle, and 22 is a dichroic mirror for reflecting YAG laser beam and part of visible semiconductor laser beam and for transmitting aiming light. The laser beam reflected by the dichroic mirror 22 is converged and focused to a diseased tissue of a patient's eye (E) by using an objective lens 23 and a contact lens 24.

Reference numeral 25 designates a slit projection optics, and the bundle through which illuminates the patient's eye E through the contact lens 24. 26 refers to as a binocular microscope for an operator to observe the patient's eye E.

### (Electrical System)

Reference numeral 30 designates a power supply for supplying energy to the flash lamp 3. The power supply 30 adjusts supplying energy in accordance with a signal from a controller 31 controlling the operation of the apparatus itself so as to vary the energy of light induced from the flash lamp 3 to the Nd:YAG rod 2. The power supply 30 also incorporates a driver for transmitting a plurality of pulses for one trigger signal.

Reference numeral 32 is a detector circuit for processing a signal from the light detector 11, 33 is another detector for processing a signal from the light detector 15. Signals processed by these detectors 32 and 33 are transmitted to the controller 31. 34 is a motor for driving the half-wavelength plate 12, and 35 is a motor driver circuit.

Reference numeral 36 is a motor for driving the safety shutter 16 to open and close in accordance with a control signal from the controller 31 and 37 is its motor driver circuit.

Reference numeral 38 designates a sensor for detecting ambient temperature surrounding the apparatus. The sensor 38 outputs a voltage signal corresponding to the temperature, which is processed in a predetermined way in the detector 39 for being input into the controller 31. Reference numeral 40 is a detector for the temperature sensor 7 in the YAG laser oscillator 1.

Reference numeral 41 designates a control panel comprising various input switches and indicators. Among the input switches, there are, for example, one for determining various irradiation conditions such as determining of irradiation energy for a diseased site as well as determining of the number of emitted pulses for one trigger signal, and one for opening the safety shutter 16 to enable YAG laser beam to be irradiated. The indicators indicate the amount of energy of the YAG laser beam detected by the detector 15, and the predetermined number of pulses. Reference numeral 42 designates an irradiation switch for generating a trigger signal for laser beam.

In the operation, an apparatus having a structure as mentioned above will be described below.

The apparatus starts up when its power switch is turned on. The apparatus is preconditioned so as to obtain a given laser output power under conditions of standard temperature. When a starting up signal is entered, the controller 31 may compensate for the energy level to be supplied to the flash lamp 3 from the power supply 30.

The controller 31 includes a program which determine the changes in the amount of supplying energy corresponding to the changes in temperature for compensating for the supplying energy to the flash lamp 3 by the detection of the changes in temperature. Temperature value in the YAG laser oscillator 1 is detected by the temperature sensor 7, and processed by the program for compensation of the energy level supplied to the flash lamp 3. The compensation is performed any time, or with a given interval of time.

The temperature sensor 38 detects the ambient temperature of the apparatus, and monitors the temperature of the environment that the apparatus is in, such that it alerts if temperature is higher than a predetermined threshold, and it determine whether the extremely high temperature value is caused by a factor within the YAG laser oscillator 1 in order to alert and instruct the operator to take an appropriate procedure. This may result in notifying the operator of the most suitable measure for such changes in as increased temperature within the apparatus and ambient temperature. The temperature value detected by the sensor 38 may be used for data for the compensation process at the time of starting up.

The controller 31 automatically generates a trigger signal when the safety shutter 16 is closed to flash the flash lamp 3 through the power supply 30 to perform an oscillation test of the YAG laser oscillator 1. The light detector 11 may detect the amount of energy of the laser beam emitted from the YAG laser oscillator 1 to determine whether or not the YAG laser oscillator 1 itself is somewhat aged. The controller 31 may also compare the detection signal from the light detector 15 with the signal from the light detector 11 to determine whether the half-wavelength plate 12 and the polarizing filter 13 operate appropriately.

The controller 31 further compensates the energy level supplied from the power supply 30 to the flash lamp 3 based on the signal output of the light detector 11 (and 15) to fine tune the energy to the flash lamp 3 so as to obtain a predetermined laser output.

The operator adjusts the irradiation conditions for the purpose of treatment by setting at an operation console 41. If irradiation energy is varied, then the controller 31 rotates the half-wavelength plate 12 by a predetermined angle based on the transmittance data of the half-wavelength plate 12 to obtain a predetermined irradiation energy. Another oscillation test is performed at this condition. The oscillation test is also performed in case of alteration of the number of irradiation pulses in the burst mode. The controller 31 automatically generates a trigger signal to perform an oscillation test of laser beam. When an oscillation test of laser beam is performed, the controller 31 determines again whether or not a predetermined amount of energy is emitted by means of the detected signal from the light detector 11 and the light detector 15. If energy is not attained to a predetermined level, then the rotation angle of the half-wavelength plate 12 will be corrected again.

In the burst mode, the number of irradiation pulses is counted by the detected signal of the light detector 11 (or 15). If the counted number of pulses is not equal to a predetermined number, then the energy level supplied to the flash lamp 3 from the power supply 30 will be further compensated. If the number surpasses the compensation limit then the operator is alerted to that.

As have been described, the apparatus performs self diagnosis each time for starting up and alteration of irradiation conditions to define the most appropriate energy of induced light of the flash lamp 3.

When the irradiation condition setting is completed, the operator pushes a switch for opening the safety shutter 16 to enable laser beam to be irradiated to a patient's eye. The operator sights the aiming light from the visible light semiconductor laser 19 to the diseased area, while observing the patient's eye through the binocular microscope 26. When the aiming is completed, then the operator may push the irradiation switch 42 to irradiate YAG laser beam to the diseased tissue.

The forgoing description of the preferred embodiments of the invention has been presented for purposes of illustration and description. It is not intended to be exhaustive or to limit the invention to the precise form disclosed, and modifications and variations are possible in the light of the above teachings or may be acquired from practice of the invention. The embodiments chosen and described in order to explain the principles of the present invention and its practical application to enable one skilled in the art to utilize the present invention in various embodiments and with various modifications as are suited to the particular use contemplated. It is intended that the scope of the present invention be defined by the claims appended hereto, and their equivalents.

## Claims

1. An apparatus for laser treatment, comprising:
a solid state laser oscillator having a Q switch, responsive to inducing light from an exciting light source for oscillating pulsed laser beam for treatment;
a power supply source for supplying variable energy to said exciting light source;
guide optics for guiding into a diseased site the pulsed laser beam emitted from the solid state laser oscillator;
pulsed output detector means for counting the number of output pulses by detecting the pulsed laser beam emitted from said solid state laser oscillator by using a light detector; and
controller means for controlling the amount of energy supplied from said power supply to the exciting light source in accordance with the number of pulses of the pulsed laser beam detected by said detector means such that a predetermined number of pulsed laser beam is emitted.

2. An apparatus for laser treatment according to claim 1, further comprising:
temperature detecting means for detecting ambient temperature of said solid state laser oscillator; and
first compensator means for compensating for the amount of energy of inducing light being controlled by said controller means in accordance with the ambient temperature value detected by said temperature detecting means.

3. An apparatus for laser treatment according to claim 2, further comprising:
attenuator means placed along with said guiding optics, for attenuating the pulsed laser beam emitted from said solid state laser oscillator;
attenuated output detecting means for detecting laser energy before and after the attenuation of said pulsed laser beam to be attenuated; and
second compensator means for compensating for the amount of energy of inducing light being controlled by said controller means by comparing pulsed laser energy before attenuation with that after the attenuation, both output energy being detected by said attenuated output detecting means.

4. An apparatus for laser treatment according to claim 1, further comprising:
attenuator means placed along with said guiding optics, for attenuating the pulsed laser beam emitted from said solid state laser oscillator;
attenuated output detecting means for detecting laser energy before and after the attenuation of said pulsed laser beam to be attenuated; and
second compensator means for compensating for the amount of energy of inducing light being controlled by said controller means by comparing pulsed laser energy before the attenuation with that after the attenuation, both output energy being detected by said attenuated output detecting means.

5. An apparatus for laser treatment according to claim 4, further comprising:
shutter means placed along with said guiding optics, for shutting up the pulsed laser beam emitted from said solid state laser oscillator;
oscillating test means for detecting output energy before and after the attenuation by the attenuator means of the pulsed laser beam emitted from said solid state laser oscillator with said shutter means being closed for performing examination of the functionality of said attenuator means.

6. An apparatus for laser treatment according to claim 1, wherein said Q switch is a Dye-Q switch.

7. An apparatus for laser treatment according to claim 1, further comprising:
shutter means placed along with said guiding optics, for shutting up the pulsed laser beam emitted from said solid state laser oscillator;
oscillating test means for detecting the output energy of the pulsed laser beam emitted from said solid state laser oscillator with said shutter means being closed for performing examination of the deterioration of the solid state laser oscillator.

8. An apparatus for laser treatment according to claim 1, further comprising:
irradiation condition setting means for presetting conditions of irradiation of pulsed laser beam emitted from said solid state laser oscillator onto a diseased site.

9. An apparatus for laser treatment, comprising:
a solid state laser oscillator responsive to inducing light from an exciting light source for oscillating pulsed laser beam for treatment;
a power supply source for supplying energy to said exciting light source;
detector means for detecting the output energy of pulsed laser beam emitted from said solid state laser oscillator;
controlling means for controlling energy supplied to said exciting light source on the basis of the results of the detection by said detector means; and
guide optics for guiding pulsed laser beam to a diseased site.

10. An apparatus for laser treatment according to claim 9, further comprising:
temperature detecting means for detecting at least one of the temperature within said solid state laser oscillator and the temperature of the surrounding ambient of the apparatus, said controlling means controlling energy supplied to said exciting light source in accordance with the results of the detection by said temperature detecting means.

11. An apparatus for laser treatment according to claim 9, wherein said solid state laser oscillator is a solid state laser oscillator having a Q switch.

12. An apparatus for laser treatment according to claim 9, further comprising:
irradiation condition setting means for presetting condition of irradiation of pulsed laser beam; and
oscillating test means for testing oscillation of pulsed laser beam from said laser oscillator at least either at the time of starting up of the apparatus or at the time of condition presetting with said irradiation condition setting means.

13. An apparatus for laser treatment according to claim 9, further comprising:
attenuator means for attenuating pulsed laser beam, said detector means having two set of detecting means for respectively detecting the laser output energy in said attenuator means at the side of the solid state laser oscillator and at the side of an eye to be operated.
